(19) Europäisches Patentamt
European Patent Office
Office européen des brevets

(11) Veröffentlichungsnummer: **0 056 589**
**A1**

(12) **EUROPÄISCHE PATENTANMELDUNG**

(21) Anmeldenummer: 82100105.4

(22) Anmeldetag: 09.01.82

(51) Int. Cl.³: C 07 C 103/42
C 07 C 103/80, C 07 D 307/68
C 07 D 233/56, C 07 D 249/08
A 01 N 37/22, A 01 N 43/08

(30) Priorität: 21.01.81 DE 3101785

(43) Veröffentlichungstag der Anmeldung:
28.07.82 Patentblatt 82/30

(84) Benannte Vertragsstaaten:
AT BE CH DE FR GB IT LI NL SE

(71) Anmelder: BAYER AG
Zentralbereich Patente, Marken und Lizenzen
D-5090 Leverkusen 1, Bayerwerk(DE)

(72) Erfinder: Lunkenheimer, Winfried, Dr.
Bismarckstrasse 29
D-5600 Wuppertal 1(DE)

(72) Erfinder: Stetter, Jörg, Dr.
Gellertweg 4
D-5600 Wuppertal 1(DE)

(72) Erfinder: Brandes, Wilhelm, Dr.
Eichendorffstrasse 3
D-5653 Leichlingen(DE)

(54) **N-substituierte Anilide, Verfahren zu ihrer Herstellung sowie ihre Verwendung als Fungizide.**

(57) Die Erfindung betrifft neue N-substituierte Anilide der allgemeinen Formel

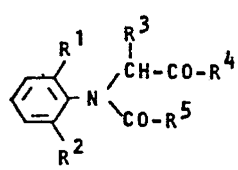

(1)

in welcher
$R^1$ für Alkyl oder Halogen steht,
$R^2$ für Wasserstoff oder Alkyl steht,
$R^3$ für Wasserstoff und Alkyl steht,
$R^4$ für für Alkyl steht,
$R^5$ für Furyl, Halogenalkyl, Cycloalkyl; sowie die Gruppierungen $-CH_2Az$, $-CH_2-OR^6$, $-CH_2-SR^6$, $-CH_2-SO_2-R^6$, $-CH_2-SO-R^6$, $-CH_2-O-SO_2R^6$, $-COOR^6$ und gegebenenfalls substituiertes Phenyl steht, wobei
$R^6$ für Alkyl, Alkenyl, Alkinyl und Alkoxyalkyl steht, und
Az für Pyrazol-1-yl, 1,2,4-Triazol-1-yl und Imidazol-1-yl steht.

mehrere Verfahren zu ihrer Herstellung und ihre Verwendung als Fungizide.

EP 0 056 589 A1

0056589



BAYER AKTIENGESELLSCHAFT
Zentralbereich
Patente, Marken und Lizenzen

5090 Leverkusen, Bayerwerk
Slr/W
I b

# N-substituierte Anilide, Verfahren zu ihrer Herstellung sowie ihre Verwendung als Fungizide

Die vorliegende Erfindung betrifft neue N-substituierte Anilide, mehrere Verfahren zu ihrer Herstellung und ihre Verwendung als Fungizide.

Es ist bereits bekannt geworden, daß Halogenacetanilide, wie beispielsweise N-Chloracetyl-N-(2-ethyl-6-methylphenyl)-alaninmethylester, mit gutem Erfolg zur Bekämpfung von pilzlichen Pflanzenkrankheiten eingesetzt werden können (vergleiche DE-OS 2 35o 944). Deren Wirkung ist jedoch, insbesondere bei niedrigen Aufwandmengen und -konzentrationen, insbesondere auch bei der Bekämpfung von Phytophthora-Arten, nicht immer ganz befriedigend.

Es wurden neue N-substituierte Anilide der allgemeinen Formel

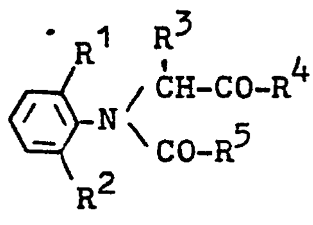

(I)

gefunden,

in welcher

$R^1$ für Alkyl oder Halogen steht,

$R^2$ für Wasserstoff oder Alkyl steht,

$R^3$ für Wasserstoff und Alkyl steht,

$R^4$ für Alkyl steht,

$R^5$ für Furyl, Halogenalkyl, Cycloalkyl; sowie die Gruppierungen $-CH_2Az$, $-CH_2-OR^6$, $-CH_2-SR^6$, $-CH_2-SO_2-R^6$, $-CH_2-SO-R^6$, $-CH_2-O-SO_2R^6$, $-COOR^6$ und gegebenenfalls substituiertes Phenyl steht, wobei

$R^6$ für Alkyl, Alkenyl, Alkinyl und Alkoxyalkyl steht, und

Az für Pyrazol-1-yl, 1,2,4-Triazol-1-yl und Imidazol-1-yl steht.

Weiterhin wurde gefunden, daß man die N-substituierten Anilide der allgemeinen Formel (I) erhält, wenn man

a) Anilide der Formel

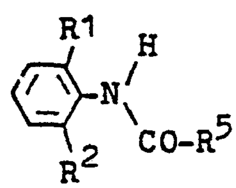

(II)

in welcher

$R^1$, $R^2$ und $R^5$ die oben angegebenen Bedeutungen haben,

mit Keto - Derivaten der Formel

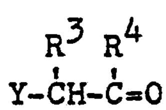

(III)

in welcher

$R^3$ und $R^4$ die oben angegebenen Bedeutungen haben und

Y für Halogen, den Mesylat- (Methylsulfonyl-) oder Tosylat- (p-Toluolsulfonyl-) Rest steht

in Gegenwart eines Säurebinders und in Gegenwart eines Verdünnungsmittels umsetzt, oder

b) Anilin-Derivate der Formel

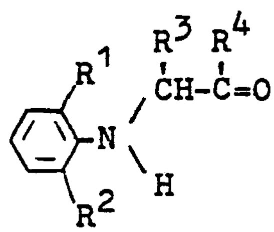

(IV)

in welcher

$R^1$, $R^2$, $R^3$ und $R^4$ die oben angegebenen Bedeutungen haben,

mit bekannten Säurehalogeniden der Formel

$$R^5\text{-CO-Hal} \qquad (V)$$

in welcher

$R^5$ die oben angegebene Bedeutung hat und

Hal für Halogen steht,

in Gegenwart eines Verdünnungsmittels und gegebenenfalls in Gegenwart eines Säurebindemittels umsetzt, oder

c) Propargyl-acetanilide der Formel

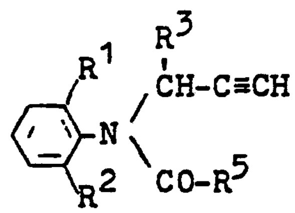

(VI)

in welcher

$R^1$, $R^2$, $R^3$ und $R^5$ die oben angegebenen Bedeutungen haben,

in üblicher Weise unter Säure-Katalyse in Gegenwart eines Schwermetallsalzes hydratisiert.

Die neuen N-substituierten Anilide weisen stark fungizide Eigenschaften auf. Dabei zeigen überraschenderweise die erfindungsgemäßen Verbindungen eine erheblich höhere Wirkung als der aus dem Stand der Technik bekannte N-Chloracetyl-N-(2-ethyl-6-methyl-phenyl)-alaninester, welcher chemisch und wirkungsmäßig eine naheliegende Verbindung ist. Die erfindungsgemäßen Stoffe stellen somit eine Bereicherung der Technik dar.

Die erfindungsgemäßen N-substituierten-Anilide sind durch die Formel (I) allgemein definiert.

In dieser Formel steht $R^1$ vorzugsweise für geradkettiges oder verzweigtes Alkyl mit 1 bis 4 Kohlenstoffatomen oder für Halogen, wie insbesondere Fluor, Chlor oder Brom. $R^2$ und $R^3$ stehen vorzugsweise für Wasserstoff, geradkettiges oder verzweigtes Alkyl mit 1 bis 4 Kohlenstoffatomen. $R^4$ steht vorzugsweise für geradkettiges oder

verzweigtes Alkyl mit 1 bis 4 Kohlenstoffatomen. $\underline{R^5}$ steht vorzugsweise für 2-Furyl, Halogenalkyl mit 1 bis 2 Kohlenstoffatomen und 1 bis 5 Halogenatomen, wobei als Halogenatome vorzugsweise Fluor und Chlor genannt seien und Cycloalkyl mit 3 bis 7 Kohlenstoffatomen, sowie für die Gruppierungen $-CH_2Az$, $-CH_2OR^6$, $-CH_2-SR^6$, $-CH_2-SO_2-R^6$, $-CH_2-SO-R^6$, $-CH_2-O-SO_2-R^6$, $-COOR^6$ und gegebenenfalls substituiertes Phenyl, wobei als Substituenten vorzugsweise infrage kommen:
Halogen, geradkettiges oder verzweigtes Alkyl mit 1 bis 4 Kohlenstoffatomen, Alkoxy mit 1 bis 2 Kohlenstoffatomen, Alkoxyalkyl mit 1 bis 4 Kohlenstoffatomen im Alkylteil und 1 bis 4 Kohlenstoffatomen im Alkoxyteil, sowie die Nitro-Gruppe. $\underline{R^6}$ steht vorzugsweise für Alkyl mit 1 bis 4 Kohlenstoffatomen, für Alkenyl und Alkinyl mit jeweils 2 bis 4 Kohlenstoffatomen und für Alkoxyalkyl mit 1 bis 4 Kohlenstoffatomen in jedem Alkylteil. $\underline{Az}$ steht dabei vorzugsweise für Pyrazol-1-yl, 1,2,4-Triazol-1-yl und Imidazol-1-yl.

Ganz besonders bevorzugt sind diejenigen N-substituierten Anilide der Formel (I), in denen $\underline{R^1}$ und $\underline{R^2}$ für Methyl, Ethyl, Isopropyl, sek-Butyl und tert-Butyl stehen; $\underline{R^1}$ außerdem für Chlor oder Brom steht; $\underline{R^2}$ außerdem für Wasserstoff steht; $\underline{R^3}$ für Methyl, Ethyl oder Wasserstoff steht; $\underline{R^4}$ für Methyl oder Ethyl steht; $\underline{R^5}$ für 2-Furyl, Methoxymethyl, Ethoxymethyl, Allyloxymethyl, Propargyloxymethyl, Ethoxymethoxymethyl, Methylmercaptomethyl, Methylsulfonyloxymethyl, Methylsulfonylmethyl, Methoxycarbonyl, Ethoxycarbonyl, Dichlormethyl, Cyclopropyl, Cyclohexyl, Pyrazol-1-yl-methyl, Imidazol-1-yl-methyl und 1,2,4-Triazol-1-yl-methyl steht.

Im einzelnen seien außer den bei den Herstellungsbeispielen genannten Verbindungen die folgenden Verbindungen der allgemeinen Formel (I) genannt:

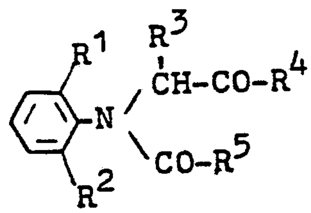

(I)

| $R^1$ | $R^2$ | $R^3$ | $R^4$ | $R^5$ |
|---|---|---|---|---|
| $CH_3$ | $CH_3$ | $CH_3$ | $CH_3$ | 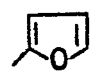 |
| $CH_3$ | $CH_3$ | $CH_3$ | $CH_3$ | $-CH_2-$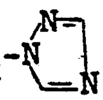 |
| $CH_3$ | $CH_3$ | $CH_3$ | $CH_3$ | $-CH_2-$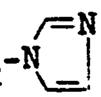 |
| $CH_3$ | $CH_3$ | $CH_3$ | $C_2H_5$ | $-CH_2OCH_3$ |
| $CH_3$ | $CH_3$ | $CH_3$ | $C_2H_5$ | 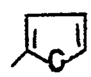 |
| $CH_3$ | $CH_3$ | $CH_3$ | $C_2H_5$ | $-CHCl_2$ |
| $CH_3$ | $CH_3$ | $CH_3$ | $C_2H_5$ | $-CO-OCH_3$ |
| $CH_3$ | $CH_3$ | $CH_3$ | $C_2H_5$ | $-CH_2-$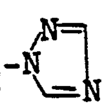 |
| $CH_3$ | $CH_3$ | $CH_3$ | $C_2H_5$ | $-CH_2-$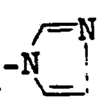 |

| $R^1$ | $R^2$ | $R^3$ | $R^4$ | $R^5$ |
|---|---|---|---|---|
| $CH_3$ | $CH_3$ | $CH_3$ | $C_3H_7$-n | $-CHCl_2$ |
| $CH_3$ | $CH_3$ | $CH_3$ | $C_3H_7$-n | $-CH_2OCH_3$ |
| $CH_3$ | $CH_3$ | $CH_3$ | $C_3H_7$-n | 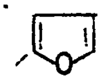 |
| $CH_3$ | $CH_3$ | $CH_3$ | $C_3H_7$-n | $-CO-OCH_3$ |
| $CH_3$ | $CH_3$ | $CH_3$ | $C_3H_7$-n | $-CH_2-$ 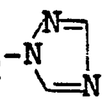 |
| $CH_3$ | $CH_3$ | $CH_3$ | $C_3H_7$-n | $-CH_2-$ 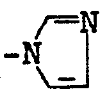 |
| $CH_3$ | $CH_3$ | $CH_3$ | $C_4H_9$-n | $-CH_2OCH_3$ |
| $CH_3$ | $CH_3$ | $CH_3$ | $C_4H_9$-n | 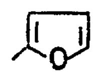 |
| $CH_3$ | $CH_3$ | $CH_3$ | $C_4H_9$-n | $-CHCl_2$ |
| $CH_3$ | $CH_3$ | $CH_3$ | $C_4H_9$-n | $-CO-OCH_3$ |
| $CH_3$ | $CH_3$ | $CH_3$ | $C_4H_9$-n | $-CH_2-$ 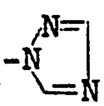 |
| $CH_3$ | $CH_3$ | $CH_3$ | $C_4H_9$-n | $-CH_2-$ 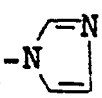 |

| $R^1$ | $R^2$ | $R^3$ | $R^4$ | $R^5$ |
|---|---|---|---|---|
| $C_2H_5$ | $C_2H_5$ | $CH_3$ | $CH_3$ | $-CH_2OCH_3$ |
| Cl | $CH_3$ | $CH_3$ | $CH_3$ | $-CH_2OCH_3$ |
| Cl | $CH_3$ | $CH_3$ | $CH_3$ | 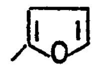 |
| $CH_3$ | $CH_3$ | $CH_3$ | $CH_3$ | $-CH_2OCH_2-CH=CH_2$ |
| $CH_3$ | $CH_3$ | $CH_3$ | $CH_3$ | $-CH_2-OCH_2-C\equiv CH$ |
| $CH_3$ | $CH_3$ | $CH_3$ | $CH_3$ | $-CH_2-O-SO_2-CH_3$ |
| $CH_3$ | $CH_3$ | $CH_3$ | $CH_3$ | $-CH_2-SO_2-CH_3$ |
| $CH_3$ | $CH_3$ | H | $CH_3$ | $-CH_2-$ 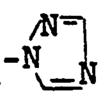 |
| $CH_3$ | $CH_3$ | H | $CH_3$ | $-CH_2-$ 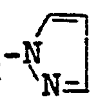 |
| $CH_3$ | $CH_3$ | H | $CH_3$ | $-CO-OCH_3$ |
| $CH_3$ | $CH_3$ | H | $CH_3$ | $-CHCl_2$ |

Verwendet man beispielsweise 2,6-Dimethyl-N-(2-furoyl)-anilin und 3-Chlor-2-butanon als Ausgangsstoffe, so kann der Reaktionsablauf durch das folgende Formelschema wiedergegeben werden (Verfahren a):

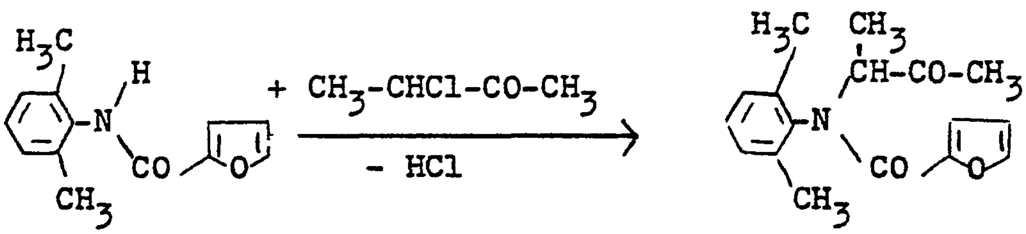


Verwendet man beispielsweise 2,6-Dimethyl-N-(1'-acetyl-ethyl)-anilin und Methoxyessigsäurechlorid als Ausgangsstoffe, so kann der Reaktionsablauf durch das folgende Formelschema wiedergegeben werden (Verfahren b):

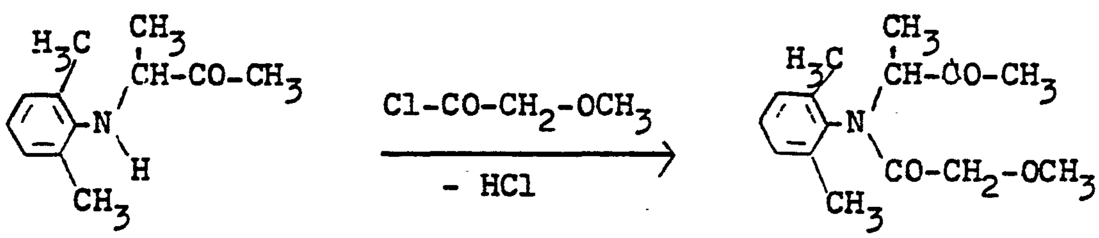


Verwendet man beispielsweise 2',6'-Dimethyl-2-methoxy-N-(1-methyl-propargyl)-acetanilid als Ausgangsstoff, so kann der Reaktionsablauf durch das folgende Formelschema wiedergegeben werden (Verfahren c):

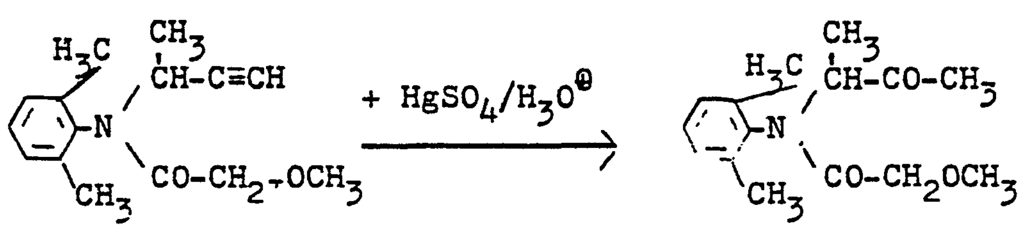

Die bei der Durchführung des erfindungsgemäßen Verfahrens (a) als Ausgangsstoffe benötigten Anilide der Formel (II) sind bekannt (vergleiche DE-OS 2 922 759 (Le A 19 685)). Sie können erhalten werden, indem man z.B. Aniline der Formel

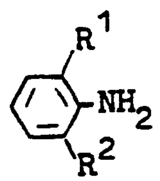

(VII)

in welcher

$R^1$ und $R^2$ die oben angegebenen Bedeutungen haben,

mit einem Säurehalogenid der Formel (V) in Gegenwart eines inerten organischen Lösungsmittels, wie beispielsweise Toluol oder Methylenchlorid, gegebenenfalls in Gegenwart eines Säurebindemittels, wie z.B. Kaliumcarbonat oder Triethylamin, und/oder in Gegenwart eines Katalysators, wie z.B. Dimethylformamid, bei Temperaturen zwischen 0 und 1oo°C umsetzt.

Die als Ausgangsstoffe benötigten Aniline der Formel (VII) sind allgemein bekannte Verbindungen der organischen Chemie. Als Beispiele seien genannt:
Anilin, 2-Methylanilin, 2-Ethylanilin, 2-Isopropylanilin, 2-sek-Butylanilin, 2-tert-Butylanilin, 2,6-Dimethylanilin, 2,3-Dimethylanilin, 2,5-Dimethylanilin, 3,5-Dimethylanilin, 2,6-Diethylanilin, 2-Ethyl-6-methylanilin, 2,3,4-Trimethylanilin, 2,4,6-Trimethylanilin, 2,4,5-Trimethylanilin, 2-Ethyl-4,6-dimethylanilin, 2,6-Diethyl-4-methylanilin, 2,6-Diisopropyl-4-methylanilin, 2,3,5-Trimethylanilin, 2,3,6-Trimethylanilin, 6-Chlor-2-methyl-anilin, 2-Brom-6-methylanilin, 2-Chlor-6-tert-butylanilin.

Die außerdem für das erfindungsgemäße Verfahren (a) als Ausgangsstoffe zu verwendenden Ketoderivate der Formel (III) sind bekannt. Sie können in allgemein bekannter Art und Weise erhalten werden, indem man z.B. entsprechende Carbonylverbindungen in üblicher Weise halogeniert.

Die bei der Durchführung des erfindungsgemäßen Verfahrens (b) als Ausgangsstoffe zu verwendenden Anilin-Derivate der Formel (IV) sind bekannt. Sie können in allgemein bekannter Art und Weise erhalten werden, indem man z.B. entsprechende Aniline mit Keto-Derivaten der Formel (III) umsetzt.

Die bei der Durchführung des erfindungsgemäßen Verfahrens (c) zu verwendenden Propargyl-acetanilide der Formel (VI) sind bekannt (vergleiche DE-OS 2 847 287 (Le A 19 217)). Die N-Propargylacetanilide der Formel (VI) können nach den dort beschriebenen Verfahren erhalten werden, indem man entweder entsprechende Propargylaniline mit Säurehalogeniden der Formel (V) oder Aniliden der Formel (II) mit entsprechenden Propargylhalogeniden in üblicher Weise umsetzt.

Die Säurehalogenide der Formel (V) sind allgemein bekannte Verbindungen der organischen Chemie.

Bei der Herstellung der neuen N-substituierten Acetanilide der Formel (I) nach den Verfahren (a), (b) und (c) können als Säurebindemittel alle üblichen Säureakzeptoren verwendet werden. Vorzugsweise in Betracht kommen Alkalicarbonate, wie Kalium- oder Natriumcarbonat.

Als Verdünnungsmittel können bei den Verfahren (a), (b) und (c) alle üblichen inerten organischen Lösungsmittel

eingesetzt werden. Vorzugsweise in Betracht kommen aromatische Lösungsmittel, wie insbesondere Toluol, Dimethylformamid und Ether, wie insbesondere Dioxan.

Nach einer bevorzugten Ausführungsform wird die Umsetzung gemäß Verfahren (a) und (b) in einem Zweiphasensystem, wie beispielsweise wässrige Natron- oder Kalilauge/Toluol oder Methylenchlorid, gegebenenfalls unter Zusatz von o,1-1 Mol eines Phasentransferkatalysators, wie beispielsweise einer Ammonium- oder Phosphoniumverbindung, durchgeführt.

Die Hydratisierung gemäß Verfahren (c) wird bevorzugt in Gegenwart von Quecksilber-II-Salzen, wie z.B. Quecksilbersulfat unter Säure-Katalyse, mit Säuren wie z.B. Schwefelsäure oder Ameisensäure, durchgeführt.

Die Reaktionstemperaturen können bei den Verfahren (a), (b) und (c) innerhalb eines größeren Bereiches variiert werden. Im allgemeinen arbeitet man zwischen 0 und 18o°C, vorzugsweise zwischen 2o und 16o°C.

Bei der Durchführung gemäß Verfahren (a) und (b) arbeitet man vorzugsweise in äquimolaren Mengen. Die Aufarbeitung und Isolierung der Reaktionsprodukte erfolgen nach üblichen Methoden.

Bei der Durchführung gemäß Verfahren (c) setzt man vorzugsweise auf 1 Mol Propargylacetanilid der Formel (VI) o,1 bis zu o,6 Mol Säure und katalytische Mengen Schwermetall-Katalysator ein. Die Aufarbeitung und Isolierung der Reaktionsprodukte erfolgen nach üblichen Methoden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Oomyceten, z.B. gegen den Erreger der Kraut- und Braunfäule der Tomate (Phytophthora infestans) eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-,Azol-u.Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

In den nachfolgenden Beispielen wird die nachstehend angegebene Verbindung als Vergleichssubstanz eingesetzt:

(A) 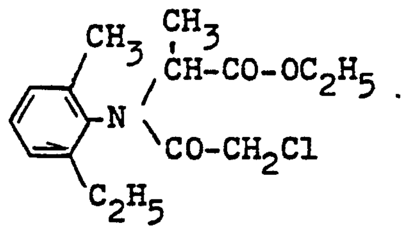

N-Chloracetyl-N-(2-ethyl-6-methylphenyl)-alaninethylester.

Beispiel A

Phytophthora-Test (Tomaten)/ Protektiv

| | | |
|---|---|---|
| Lösungsmittel: | 4,7 Gewichtsteile | Aceton |
| Emulgator : | 0,3 Gewichtsteile | Alkyl-aryl-polyglykolether |
| Wasser : | 95,0 Gewichtsteile | |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.
Mit der Spritzflüssigkeit bespritzt man junge Tomatenpflanzen mit 2 bis 4 Laubblättern bis zur Tropfnässe. Die Pflanzen verbleiben 24 Stunden bei 20°C und einer relativen Luftfeuchtigkeit von 70% im Gewächshaus. Anschließend werden die Tomatenpflanzen mit einer wässrigen Sporensuspension von Phytophthora infestans inokuliert. Die Pflanzen werden in eine Feuchtkammer mit einer 100%igen Luftfeuchtigkeit und einer Temperatur von 18 bis 20°C gebracht.
Nach 5 Tagen wird der Befall der Tomatenpflanzen bestimmt. Die erhaltenen Boniturwerte werden auf Prozent Befall umgerechnet. 0 % bedeutet keinen Befall, 100 % bedeutet, daß die Pflanzen vollständig befallen sind.
In diesem Test zeigen z.B. folgende Verbindungen eine sehr gute Wirkung, die derjenigen der aus dem Stand der Technik bekannten Verbindung(A)deutlich überlegen ist:

Verbindungen gemäß Herstellungsbeispielen 1, 2, 3 und 4.

Herstellungsbeispiele

Beispiel 1:

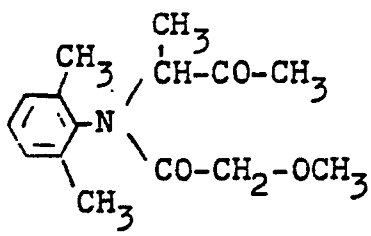

(Verfahren c)

In eine Lösung von 2,67 g (o,ooo9 Mol) Quecksilber(II)-sulfat und 9,18 g (o,o9 Mol) konz. Schwefelsäure in 3oo ml Wasser tropft man bei 6o°C eine Lösung von 73,5 g (o,3 Mol) 2',6'-Dimethyl-2-methoxy-N-(1-methylpropargyl)-acetanilid in 15o ml Dioxan und rührt 3,5-Stunden bei 6o bis 65°C. Nach Abkühlung auf 2o°C wird mehrmals mit Ether extrahiert. Die organische Phase wird mit Natriumhydrogencarbonatlösung und mit Wasser gewaschen, über Natriumsulfat getrocknet und eingeengt. Man erhält 63,4g (8o,2% der Theorie) 2',6'-Dimethyl-2-methoxy-N-(1-acetylethyl)-acetanilid als braunes Öl mit dem Brechungsindex $n_D^{2o}$ = 1,5126.

Herstellung des Ausgangsproduktes

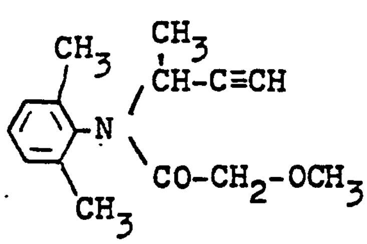

44,8 g (o,25 Mol) 3-(2,6-Dimethylanilino)-1-butin und 29,6 g (o,375 Mol) Pyridin werden in 2oo ml Tetrahydrofuran unter Rückfluß erhitzt und tropfenweise mit 41,o g

(o,375 Mol) Methoxyacetylchlorid versetzt. Es wird noch 1,5 Stunden unter Rückfluß erhitzt. Anschließend wird das Lösungsmittel abdestilliert und der Rückstand zwischen Essigester und verdünnter Salzsäure verteilt. Die organische Phase wird abgetrennt, mit 5%iger Natronlauge und Wasser gewaschen, über Natriumsulfat getrocknet und eingeengt. Der Rückstand wird in Ligroin umkristallisiert. Man erhält 47,8 g (76,5% der Theorie) 2',6'-Dimethyl-2-methoxy-N-(1-methyl-propargyl)-acetanilid vom Schmelzpunkt 73°C.

Entsprechend Beispiel 1 sowie entsprechend den Verfahrensvarianten (a) bis (c) werden die Verbindungen der allgemeinen Formel

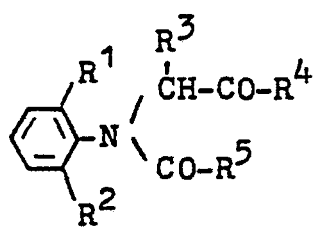

(I)

erhalten:

| Beispiel Nr. | $R^1$ | $R^2$ | $R^3$ | $R^4$ | $R^5$ | Phys. Konst. |
|---|---|---|---|---|---|---|
| 2 | $CH_3$ | $CH_3$ | $CH_3$ | $CH_3$ | $-CH_2Cl$ | 97-99°C |
| 3 | $CH_3$ | $CH_3$ | $CH_3$ | $CH_3$ | $-COOCH_3$ | 92-93°C |
| 4 | $CH_3$ | $CH_3$ | $CH_3$ | $CH_3$ | $-CHCl_2$ | 133-134°C |
| 5 | $CH_3$ | $CH_3$ | $CH_3$ | $CH_3$ | 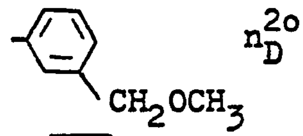 | $n_D^{20}$:1,5700 |
| 6 | $CH_3$ | $CH_3$ | H | $CH_3$ | 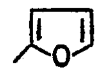 | 89-91°C |
| 7 | $CH_3$ | $CH_3$ | H | $CH_3$ | $-CH_2OCH_3$ | 59°C |

Patentansprüche

1) N-Substituierte Anilide der allgemeinen Formel

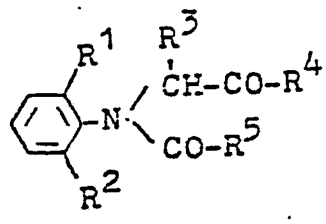

(I)

in welcher

$R^1$ für Alkyl oder Halogen steht,

$R^2$ für Wasserstoff oder Alkyl steht,

$R^3$ für Wasserstoff und Alkyl steht,

$R^4$ für für Alkyl steht,

$R^5$ für Furyl, Halogenalkyl, Cycloalkyl; sowie die Gruppierungen $-CH_2Az$, $-CH_2-OR^6$, $-CH_2-SR^6$, $-CH_2-SO_2-R^6$, $-CH_2-SO-R^6$, $-CH_2-O-SO_2R^6$, $-COOR^6$ und gegebenenfalls substituiertes Phenyl steht, wobei

$R^6$ für Alkyl, Alkenyl, Alkinyl und Alkoxyalkyl steht, und

Az für Pyrazol-1-yl, 1,2,4-Triazol-1-yl und Imidazol-1-yl steht.

2) Verbindungen der Formel (I) in Anspruch 1, wobei

$R^1$ für Methyl, Ethyl, Isopropyl, sek.-Butyl, tert.-Butyl, Chlor oder Brom steht,

$R^2$ für Wasserstoff, Methyl, Ethyl, Isopropyl, sek.-Butyl oder tert.-Butyl steht,

$R^3$ für Wasserstoff, Methyl oder Ethyl steht,

$R^4$ für Methyl oder Ethyl steht,

$R^5$ für 2-Furyl, Methoxymethyl, Ethoxymethyl, Allyloxymethyl, Propargyloxymethyl, Ethoxymethoxymethyl, Methylmercaptomethyl, Methylsulfonyloxymethyl, Methylsulfonylmethyl, Methoxycarbonyl, Ethoxycarbonyl, Dichlormethyl, Cyclopropyl, Cyclohexyl, Pyrazo -1-yl-methyl, Imidazol-1-yl-methyl und 1,2,4-Triazol-1-yl-methyl steht.

3) Verfahren zur Herstellung von N-substituierten Aniliden der allgemeinen Formel

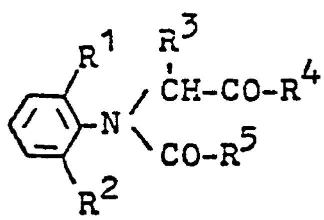

(I)

in welcher

$R^1$ für Alkyl oder Halogen steht,

$R^2$ für Wasserstoff oder Alkyl steht,

$R^3$ für Wasserstoff und Alkyl steht,

$R^4$ für Alkyl steht,

$R^5$ für Furyl, Halogenalkyl, Cycloalkyl; sowie die Gruppierungen $-CH_2Az$, $-CH_2-OR^6$, $-CH_2-SR^6$,

$-CH_2-SO_2-R^6$, $-CH_2-SO-R^6$, $-CH_2-O-SO_2R^6$, $-COOR^6$ und gegebenenfalls substituiertes Phenyl steht, wobei

$R^6$ für Alkyl, Alkenyl, Alkinyl und Alkoxyalkyl steht, und

Az für Pyrazol-1-yl, 1,2,4-Triazol-1-yl und Imidazol-1-yl steht,

dadurch gekennzeichnet, daß man

a) Anilide der Formel

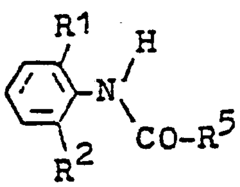

(II)

in welcher

$R^1$, $R^2$ und $R^5$ die oben angegebenen Bedeutungen haben,

mit Keto-Derivaten der Formel

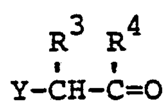

(III)

in welcher

$R^3$ und $R^4$ die oben angegebenen Bedeutungen haben und

Y für Halogen, den Mesylat- oder Tosylat-Rest steht,

in Gegenwart eines Säurebinders und in Gegenwart eines Verdünnungsmittels umsetzt, oder

b) Anilin-Derivate der Formel

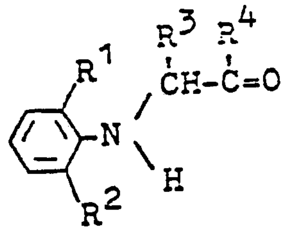

(IV)

in welcher

$R^1$,$R^2$,$R^3$ und $R^4$ die oben angegebenen Bedeutungen haben,

mit bekannten Säurehalogeniden der Formel

$R^5$-CO-Hal (V)

in welcher

$R^5$ die oben angegebene Bedeutung hat und

Hal für Halogen steht,

in Gegenwart eines Verdünnungsmittels und gegebenenfalls in Gegenwart eines Säurebindemittels umsetzt, oder

c) Propargyl-acetanilide der Formel

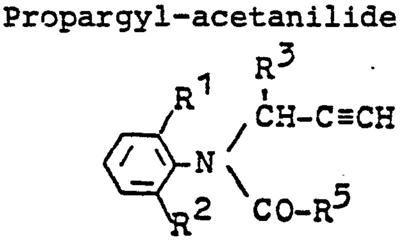

(VI)

in welcher

$R^1$,$R^2$,$R^3$ und $R^5$ die oben angegebenen Bedeutungen haben,

in üblicher Weise unter Säure-Katalyse in Gegenwart eines Schwermetallsalzes hydratisiert.

4) Fungizide Mittel, gekennzeichnet durch einen Gehalt an mindestens einem N-substituierten Anilid der Formel (I) in Ansprüchen 1 und 3.

5) Verfahren zur Bekämpfung von Pilzen dadurch gekennzeichnet, daß man N-substituierte Anilide der Formel (I) in Ansprüchen 1 und 3 auf Pilze oder ihren Lebensraum einwirken läßt.

6) Verwendung von N-substituierten Aniliden der Formel (I) in Ansprüchen 1 und 3 zur Bekämpfung von Pilzen.

7) Verfahren zur Herstellung von fungiziden Mitteln, dadurch gekennzeichnet, daß man N-substituierte Anilide der Formel (I) in Ansprüchen 1 und 3 mit Streckmitteln und/oder oberflächenaktiven Mitteln vermischt.

0056589

Europäisches Patentamt

# EUROPÄISCHER RECHERCHENBERICHT

Nummer der Anmeldung
EP 82 10 0105

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X | EP - A - 0 000 051 (BAYER)<br>* Patentansprüche * | 1,3-7 | C 07 C 103/42<br>103/80<br>C 07 D 307/68<br>C 07 D 233/56<br>C 07 D 249/08<br>A 01 N 37/22<br>43/08 |
| | -- | | |
| X | EP - A - 0 010 715 (BAYER)<br>* Patentansprüche 1-5,9-10; Seiten 55,63 * | 1 | |
| | -- | | |
| X | GB - A - 2 019 404 (CHEVRON)<br>* Patentansprüche 1,32; Seite 10 * | 1 | RECHERCHIERTE SACHGEBIETE (Int Cl.³) |
| | -- | | |
| X | EP - A - 0 019 858 (BAYER)<br>* Seite 56 * | 1-2 | C 07 C 103/00<br>149/00<br>C 07 D 307/00<br>A 01 N 37/00<br>43/00 |
| | -- | | |
| Y | DE - A - 3 025 694 (CIBA-GEIGY)<br>* Patentansprüche * | 1,4-7 | |
| | ---- | | |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 07-04-1982 | MOREAU |

EPA form 1503.1 06.78